# EUROPEAN PATENT SPECIFICATION

(11) **EP 4 360 693 B1**
(45) Date of publication and mention of the grant of the patent: **15.04.2026**
(21) Application number: 21946677.8
(22) Date of filing: 10.08.2021
(51) Int. Cl.: A61N 1/36, A61N 1/05

(54) **PERCUTANEOUS IMPLANTATION ELECTRODE**
ELEKTRODE FÜR PERKUTANE IMPLANTATION
ÉLECTRODE À IMPLANTATION PERCUTANÉE

(30) Priority: 23.06.2021 CN 202110699959
(43) Date of publication of application: 01.05.2024
(73) Proprietor: Jiangsu Ced Medtech Co., Ltd, Rudong Nantong, Jiangsu 226499 (CN)
(72) Inventor: HUANG, Baoming, Shanghai 201210 (CN); YE, Jinnan, Shanghai 201210 (CN); ZHU, Yueliang, Shanghai 201210 (CN); ZHANG, Shan, Shanghai 201210 (CN)
(74) Representative: Loo, Chi Ching
(86) International application number: PCT/CN2021/111820
(87) International publication number: WO 2022/267192

(56) References cited:
- EP-A1- 2 835 148
- CN-A- 101 502 699
- CN-A- 105 536 141
- CN-A- 106 573 141
- CN-A- 108 472 484
- CN-A- 111 265 771
- CN-A- 112 402 786
- US-A- 5 728 149
- US-A1- 2006 122 682
- US-A1- 2010 249 892
- US-A1- 2012 191 169
- US-A1- 2014 330 355
- US-A1- 2017 340 891
- US-A1- 2021 146 121

## Description

### Technical Field

The present disclosure relates to the field of medical devices, and more particularly to a percutaneous implanted lead.

### Background Art

It is well known that pain is a difficult disease to cure in humans, that the mechanism of pain is extremely complex. The electrical stimulation therapy has a long history, including implantable spinal cord stimulators, non-implantable epidermal stimulation, and peripheral nerve therapy with percutaneously implanted lead and external electrical stimulators. Although these devices reduce pain and improve the quality of life for patients, these stimulation systems have various defects and deficiencies, as well as differences in the patient's human anatomy, insufficient electrical stimulation energy (including electrode displacement), inability of electrical stimulation to cover the target of painful nerve, etc., resulting in ineffective pain suppression in patients. US patent application publication US 2017/340891 A1 discloses a percutaneous implanted lead.

Patent No. US4556051, filed in 1985, and Patent No. US5830151, filed in 1998, disclose methods and devices for the treatment of peripheral nerve electrical stimulation. Although peripheral nerve stimulation has a long history, it is not widely used because of the poor effectiveness of surface electrodes to provide electrical stimulation and the inconvenience of wearing electrical stimulators which are relatively large.

Patent No. US20180056066A1 discloses a percutaneous stimulation system in which a loop is created between a percutaneous electrode and a surface electrode so as to achieve the effect of nerve electric stimulation. However, the surface electrode in the system can only be below the stimulator. If the surface electrode is on the surface of the human skin where the stimulator is inconveniently placed, the surface electrode cannot be realized, thus reducing the range of surface electrode that can be placed. The optimal path for stimulation may not be reached, resulting in that the effect of treating nerve pain for patients is limited. Most importantly, the above patent has only a single percutaneous electrode, which can only achieve monopolar stimulation of a percutaneous electrode and stimulation of a single path. The effect of peripheral nerve stimulation is influenced by the relative position of the stimulation electrode and the target nerve. Stimulation may only occur if the target nerve is in the stimulation path. Also, if the electrode is close to the target nerve, the stimulation current easily covers the target nerve, thereby easily producing a better effect, and conversely, hardly having an effect. Therefore, the stimulation effect of the single electrode is easily influenced by the implantation position. Even if the electrode is in place at the time of implantation, the percutaneous electrodemay dislodgeover time. Additionally, a single percutaneous electrodeis not capable of bipolar or multipolar stimulation in vivo. All of the above affect the effectiveness of the single percutaneous electrode system in stimulating nerves and treating pain.

At present, many doctors implant spinal cord stimulation electrodes into peripheral nerve for pain treatment. However, the percutaneous implanted lead for spinal cord stimulation adopts the closed insulating layer which is disposed on the whole periphery after the metal wire is spirally wound. Its diameter is generally thick, ranging from 1.3 mm-1.4 mm. Therefore, the outer diameter of the needle tube of the puncture needle in which the percutaneous implanted lead is required to be implanted is also relatively bigger. It is difficult to implant it into the peripheral nerve, causing great trauma to patients and increasing the risk of infection. The clinical need for peripheral nerves is to design the percutaneous implanted lead to be 0.70 mm with smaller diameter. The design and manufacture of the percutaneous implanted lead in this size range has great challenges and difficulties. We face the problem how to design the spiral conductor wire under the limited cross-sectional area and resist fatigue distortion and bending, and the insulation between spiral conductor wires is also a great challenge.

### Summary of the disclosure

The present invention is defined by the appended claims. Aspects, embodiments and examples described hereinafter are only of exemplary nature and are presented for better understanding the present invention. The technical problem to be solved by the present disclosure is to provide a percutaneous implanted lead, which has a small outer diameter and can be implanted into the human body with a puncture needle having a smaller outer diameter of a needle tube, has good anti-fatigue twisting and bending performance, and has tiny trauma after implantation into the human body and reduces the risk of infection.

To solve the above technical problems, the disclosure provides a percutaneous implaned lead, comprising a distal lead head and a lead body, the distal lead head comprising at least one electrode, the lead body being formed by spirally winding at least one conductor wire, and the conductor wire comprising a metal wire and an insulating layer, which is arranged outside the metal wire in a wrapping manner, such that the lead body is of an open-type insulating spiral structure.

Preferably, the distal lead head comprises a first electrode and a fixation hook; the lead body is formed by spirally winding an conductor wire; spiral turns constituting the lead body are sequentially arranged at a predetermined pitch in an axial direction; the first electrode is formed in a distal direction under an exposed state by spirally winding the metal wire constituting the conductor wire; and the metal wire at the distal end of the first electrode continues to extend distally and is bent to form the fixation hook.

Preferably, the pitch of the first electrode between two adjacent turns within 2-3 turns of the distal end thereof is greater than the pitch elsewhere.

Preferably, the distal lead head comprises a first electrode and a fixation hook; wherein the first electrode is a tubular metal electrode; the lead body is formed by spirally winding an conductor wire; the spiral turns constituting the lead body are sequentially arranged at a predetermined pitch along the axial direction; the metal wire constituting the conductor wire extends distally through an electrode tube of the metal electrode under an exposed state and continues to extend and bend to form the fixation hook; and the metal wire is electrically connected with the metal electrode.

Preferably, the distal lead head comprises a first electrode and a fixation hook, wherein the first electrode is a tubular metal electrode; the lead body is formed by spirally winding the conductor wire; the spiral turns constituting the lead body are sequentially arranged at a predetermined pitch along the axial direction; a distal end of the metal wire constituting the conductor wire extends and then extends into a proximal end of the electrode tube of the first electrode and is electrically connected to the first electrode; the fixation hook is made of the metal wire; and the proximal end of the fixation hook extends into a distal end of the electrode tube of the first electrode and is connected to the first electrode.

Preferably, the distal lead head comprises a second electrode, a first electrode and a fixation hook in sequence from the proximal end to the distal end, wherein the first electrode and the second electrode are arranged along a same axial interval and insulated; the lead body is formed by spirally winding two conductor wires which are a first conductor wire and a second conductor wire, respectively; and the spiral turns constituting the lead body are sequentially arranged at a predetermined pitch along the axial direction.

Preferably, the first electrode and the second electrode are both tubular metal electrodes; the first conductor wire is insulated to pass through the electrode tube of the second electrode; the metal wire constituting the first conductor wire extends distally through the electrode tube of the first electrode and then continues to extend distally and bend to form the fixation hook, the metal wire of the first conductor wire being electrically connected to the first electrode; the exposed metal wire of the second conductor wire at the distal end extends into the electrode tube of the second electrode and is electrically connected to the second electrode; or, the metal wire constituting the second conductor wire extends distally under an exposed state and is spirally wound to form the second electrode; the distal end of the first conductor wire is insulated to pass through a cavity spirally formed by the second electrode; the metal wire constituting the first conductor wire extends distally under an exposed state and is spirally wound to form the first electrode; and the metal wire of the first electrode at the distal end continues to extend distally and is bent to form the fixation hook.

Preferably, the first conductor wire between the first electrode and the second electrode is provided with an insulating buffer sleeve.

Preferably, the bending angle of the fixation hook is 20-60 degrees.

Preferably, the metal wire has a diameter of 0.1 mm-0.15 mm, and the outer diameter of the first electrode or/and the second electrode is 0.55 mm-0.70 mm.

Preferably, the metal wire is 316L stainless steel wire or MP35N nonmagnetic nickel-cobalt-chromium-molybdenum alloy wire; the insulating material used for the insulating layer is polyurethane, teflon or ethylene-tetrafluoroethylene copolymer; and the insulating material is extruded to a tubular form and then applied to the periphery of the metal wire, or the insulating material is sprayed onto the metal wire by using a coating process.

Preferably, the exposed metal wire is welded or crimped to the first electrode or the second electrode.

The present disclosure has the following advantageous effects compared to the prior art.

According to the percutaneous implanted lead provided by the present disclosure, the lead body of the percutaneous implanted lead forms an open spiral structure after the metal wire being spirally wound and insulated, which is more conducive to the lead body to bear the traction of the muscle tissue in the body and has a better elasticity than the case where is forms a closed insulating layer around the whole periphery after the metal wire being spirally wound. Due to the open spiral structure of the lead body, the diameter of the percutaneous part of the lead body may be reduced to the diameter of a single wire plus the thickness of the wrapped insulating layer (0.20 mm-0.30 mm), rather than the diameter (0.50 mm-0.70 mm) formed by wrapping the insulating layer integrally after the spiral winding, which has less trauma and faster healing and reduces the risk of infection. In addition, it may be integrally wound with one or two metal wires with the insulating layer, so as to reduce the risk of subsequent fracture caused by processing technology in the connection process of electrical conductor and the risk of connection separation caused by fatigue and wear in clinical use. In particular, the tightly wound spiral electrode structure may increase the stimulation surface area for human body and improve the bearable stimulation current. In particular, the lead body of the percutaneous implanted lead is designed by using extremely fine double-stranded insulated metal wire in a stacked spiral shape, and a cylindrical or stacked spiral double-electrode structure and a structure fixed to the human tissue are formed at the distal end of the implantation electrode. The three electrodes composed of internal double electrodes and a surface electrode may form six different stimulation pathways composed of stimulation electrodes and return electrodes, and each stimulation pathway may play a role in different nerve tissues. Furthermore, in the case of a combination of two electrodes, by distributing the current intensity between the two electrodes, the overall flow direction of the current is controlled, reaching the effect of the virtual electrode, providing a new possibility to increase the stimulation effect.

### Brief Description of the Drawings

Figs. 1a and 1b are schematic views showing the structure of a percutaneous implanted lead having a monopolar electrode according to a first example of the present disclosure;
Fig. 2 is a schematic view showing the structure of a percutaneous implanted lead having a monopolar electrode according to a second example of the present disclosure;
Fig. 3 is a schematic view showing the structure of a percutaneous implanted lead having a bipolar electrode according to a third example of the present disclosure;
Fig. 4 is a schematic view showing the structure of a percutaneous implanted lead having a bipolar electrode according to a fourth example of the present disclosure;
Fig. 5 is a schematic view showing an overall structure of a peripheral nerve stimulation system according to an example of the present disclosure;
Figs. 6a and 6b are schematic views showing the structure of a remote controller according to an example of the present disclosure, and Fig. 6c is a schematic view of a clinical programmer;
Fig. 7a is a schematic view showing the structure of a fixed surface electrode according to an example of the present disclosure, and Fig. 7b is a schematic view showing the structure of a movable surface electrode;
Fig. 8 is a schematic view of a peripheral nerve stimulation system for a patient according to an example of the present disclosure;
Fig. 9 is a schematic circuit diagram of a stimulator according to an example of the present disclosure;
Fig. 10(a) is a schematic view of six possible stimulation paths of three electrodes, and Fig. 10(b) is a schematic view of a virtual electrode formed by different division of two electrode currents of an electrode combination and a change in the direction of the total current;
Fig. 11(a) is a schematic view of a stimulation loop formed by a stimulation electrode and a return electrode, and Fig. 11(b) is a schematic view of the operating principle of a bi-directional current pulse source;

In the drawings,
1-subcutaneous tissue, 2-wound, 3-patient, 10- percutaneous implanted lead, 11-distal lead head , 12-lead body, 13-lead tail end, 30-first cable, 31-first wiring port, 40-third cable, 41-second wiring port, 50-stimulator, 51-stimulation controller, 52-pulse generator, 521-pulse source, 53-third wiring port, 60-remote controller, 61-control key, 62-display screen, 63-hanging hole, 64-hand grip, 65-USB interface, 70-fixed surface electrode, 701-first surface patchsurface patch, 801-second surface patchsurface patch, 702, 802-hydrogel, 71-first surface electrode interface, 72-second cable, 80-movable surface electrode, 81-second surface electrode interface, 91-third surface electrode interface, 901-third surface patch, 902-adhesive, 100-clinical programmer, 111-first electrode, 112-second electrode, 113-fixed hook, 114-insulating buffer sleeve, 121-conductor wire, 122-metal wire, 123-insulating layer, 1131-bent portion, 1132-linear portion, 1211-first conductor wire, 1212-second conductor wire.

### Detailed Description of the disclosure

The present disclosure are further described below in combination with the attached drawings and examples.

In order to more clearly describe the structural features of the present disclosure, the present disclosure uses "proximal", "distal", and "axial" as directional terms, wherein "proximal" refers to an end near to the operator; "distal" refers to an end away from the operator; "distal lead head " refers to a most distal part of the percutaneous implanted lead; and "axial" refers to a direction of the central axis of the electrode or a direction parallel to the central axis of the electrode. The term "or" is generally employed in its sense including "and/or" unless the content clearly dictates otherwise.

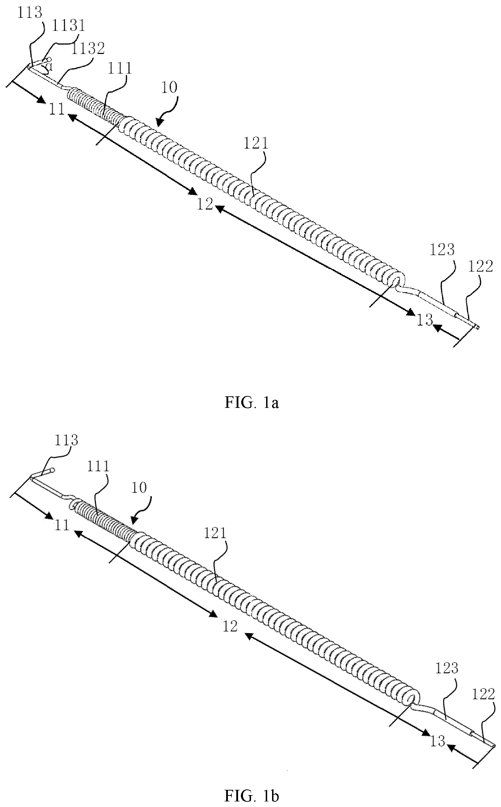

Referring to Figs. 1a-4, the present example provides a percutaneous implanted lead 10 including an distal lead head 11 and an lead body 12. The distal lead head 11 includes at least one electrode. The lead body 12 is formed by spirally winding at least one conductor wire 121. The conductor wire 121 includes a metal wire 122 and an insulating layer 123. The metal wire 122 surrounds the insulating layer 123, so that the lead body 12 is of an open-type insulating spiral structure. The spiral turns constituting the lead body 12 are sequentially arranged at a predetermined pitch along the axial direction. It is advantageous for the lead body 12 to withstand the pulling of the muscle tissue in the body, to have a more excellent elasticity, and to have a pitch size specially designed as required, preferably 0.38 mm-0.48 mm. The metal wire 122 is preferably a 316L stainless steel wire or a MP35N non-magnetic nickel-cobalt-chromium-molybdenum alloy wire. The insulating material of the insulating layer 123 is preferably polyurethane (PU), polytetrafluoro ethylene (PTFE) or ethylene-tetrafluoro ethylene copolymer (ETFE). The insulating material is extruded into a tube type and then applied to the periphery of the metal wire 122, or the insulating material is sprayed onto the metal wire 122 by using a coating process. The metal wire 122 may be a very fine metal wire. The diameter of the metal wire is preferably 0.1 mm -0.15 mm, and the diameter of the prepared percutaneous implanted lead is 0.55 mm-0.70 mm, which can meet the clinical requirements of peripheral nerve stimulation. Therefore, compared with the prior art in which the metal wire is spiraled to form a closed insulation structure at the periphery of the whole spiral structure, due to the open spiral structure of the lead body 12, the diameter of the percutaneous portion of the lead body 12 is reduced to a single wire diameter plus the thickness of the wrapped insulating layer (0.20 mm-0.30 mm), rather than the diameter formed after the the spiral winding (0.50 mm-0.70 mm), after contraction and compression of the skin wound, which results in less trauma and faster healing for the patient, reduces the risk of wound infection and provides more effective treatment for neuromodulation.

### Example 1

Referring to Fig. 1a, the percutaneous implanted lead 10 in the present example has an electrode in a monopolar electrode structure. The distal lead head 11 includes a first electrode 111 and a fixation hook 113. The lead body 12 is formed by spirally winding an conductor wire 121. The spacing between adjacent spiral turns constituting the lead body 12 is preferably 0.38 mm-0.48 mm. The first electrode 111 is formed into a spiral structure by tightly winding the metal wire 122 constituting the conductor wire 121 in a distal direction under an exposed state. The spiral turns constituting the first electrode 111 are successively fitted along the axial direction. The tightly wound structure of the first electrode 111 may increase the stimulation surface area of the percutaneous implanted lead 10. The metal wire 122 at the distal end of the first electrode 111 continues to linearly extend to the distal end and is bent to form the fixation hook 113. The bending angle α formed between the bent portion 1131 of the fixation hook 113 and the linear portion 1132 is 10°-60°, preferably 20°-60°. The fixation hook 113 is provided so as to maintain the stimulation position of the first electrode 111 during the treatment of the patient (30-90 days or longer), and reduce the dislocation of the first electrode 111 so as to improve the treatment effect. The lead body 12 may also be implanted in a patient, and the flexibility resulting from arranging the lead body 12 in a special pitch and open spiral structure may overcome muscle stretching and twisting in the patient. The open insulated spiral structure in turn may reduce the diameter of the percutaneous electrode 10 to an extreme value, effectively reducing the risk of wound infection. The percutaneous implanted lead 10 with this structure may be integrally processed as a whole. An insulating layer 123 is formed outside the metal wire 122 where insulation is required by the coating processing technology, so that it has a small diameter and high strength. Then, the distal lead head 11, the lead body 12 and the lead tail end 13 are integrally wound by the spiral winding process, which greatly reduces the risk of subsequent fracture caused by the processing technology during the connection of electrical conductors and the risk of connection separation in clinical use.

Further, as shown in Fig. 1b, in this embodiment, the pitch of the first electrode 111 between adjacent spiral coils within 2-3 turns of the distal end is greater than elsewhere. This structure is similar to that shown in Fig. 1a, but the addition of a 1-2 turn large pitch transition between the first electrode 111 and the fixation hook 113 reduces the stress concentration at this location, thereby improving the overall tensile strength at the location of the fixation hook 113.

### Example 2

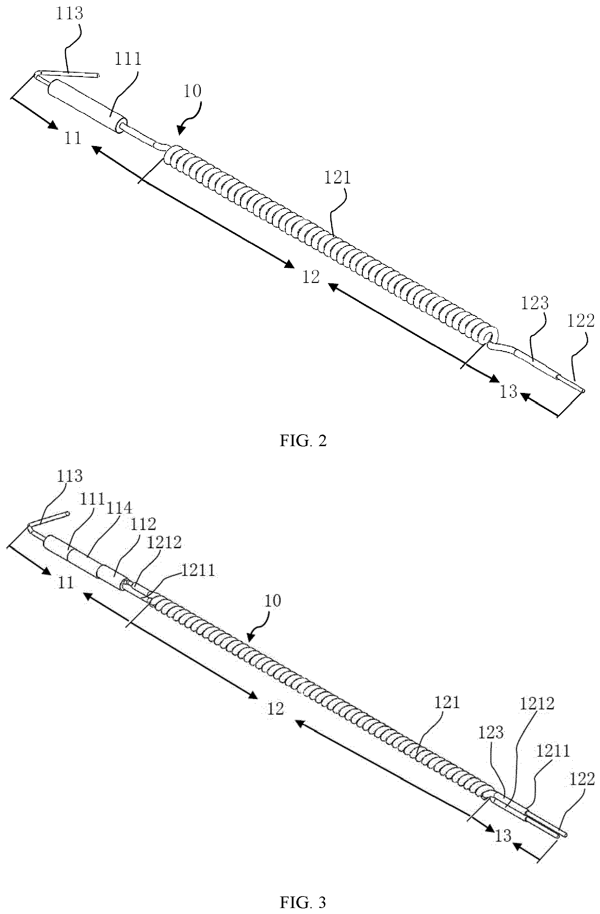

With reference to Fig. 2, the percutaneous implanted lead 10 of the present example has one electrode, which is a monopolar electrode structure. The distal lead head 11 includes a first electrode 111 and a fixation hook 113. The first electrode 111 is a tubular metal electrode. The metal electrode may be a platinum-iridium electrode which is a good implantation lead body and has better electricity and in-vivo corrosion resistance than a general metal wire. The lead body 12 is formed by spirally winding an conductor wire 121. In an embodiment, the metal wire 122 constituting the conductor wire 121 extends distally through an electrode tube of the metal electrode under an exposed state and then continues to extend and bend to form the fixation hook 113, and the metal wire 122 is electrically connected to the metal electrode. It directly uses a tubular metal electrode as the first electrode 111 in the structure, without performing a winding process requiring a higher consistency for the first electrode 111. The processing technology is relatively simple, and the first electrode 111 of the tubular structure has a stronger tensile resistance structure than that of the spiral structure. In another embodiment, the distal end of the metal wire 122 constituting the conductor wire 121 extends into the proximal end behind the electrode tube of the metal electrode and is electrically connected to the first electrode 111. The fixation hook 113 is made of another metal wire or other materials. The material of the fixation hook 113 may be the same as that of the metal wire 122, may also be a material different from that of the metal wire 122 according to needs, and may even be a non-metallic insulating material. Whether the fixation hook 113 and the first electrode 111 are selected is based on the electrical stimulation requirements. The proximal end of the fixation hook 113 extends into the distal end of the electrode tube of the metal electrode and is connected to the first electrode 111. This structure may change the material and structure of the fixation hook 113 more flexibly, thereby achieving a better fixing effect. The metal wire 122 establishes a reliable mechanical connection or a mechanical and electrical simultaneous connection with the first electrode 111 by welding, crimping or other mechanical connection means. The crimping method refers to a connecting method in which a portion of one part is inserted into another part and then both parts are fixed by pressing the combined portion. The crimping method in the present example means that the exposed metal wire 122 of the conductor wire 121 or the metal wire constituting the fixation hook 113 is inserted into the electrode tube, and then the electrode tube is squeezed to establish a reliable electrical connection between the metal wire 122 and the electrode tube.

### Example 3

With reference to Fig. 3, the percutaneous implanted lead 10 of the present example has two electrodes in a double-electrode structure. The distal lead head 11 includes, in sequence from the proximal end to the distal end, a second electrode 112, a first electrode 111 and a fixation hook 113. The first electrode 111 and the second electrode 112 are spaced apart and insulated along the same axial direction, and the spacing distance is preferably 10-15 mm. The lead body 12 is formed by spirally winding two conductor wires 121. The two conductor wires 121 are a first conductor wire 1211 and a second conductor wire 1212, respectively. The first electrode 111 is connected to the adapter 20 via the first conductor wire 1211. The second electrode 112 is connected to the adapter 20 via the second conductor wire 1212, any one electrode or a combination of any two electrodes of the first electrode 111, the second electrode 112 and the surface electrode 70 serves as a stimulation electrode or a channel electrode, and constitutes a stimulation return together with the remaining one or two electrodes. Therefore, the three electrodes of the first electrode 111, the second electrode 112 and the surface electrode may form six different stimulation pathways composed of a stimulation electrode and a returm electrode. Each stimulation pathway may have an effect on different nerve tissues, or six stimulation effects may be obtained under the same electrode implantation conditions. Furthermore, in the case of a combination of two electrodes, by distributing the current intensity between the two electrodes, the overall flow direction of the current is controlled, reaching the effect of the virtual electrode, providing a new possibility to increase the stimulation effect. Compared with the unipolar structure, the percutaneous implanted lead 10 with the bipolar structure has larger stimulation range and more stimulation modes, increases the diversity of treatment and realizes different stimulation functions and stimulation effects, which is beneficial to improve the clinical treatment effect.

With continuing reference to Fig. 3, both the first electrode 111 and the second electrode 112 are tubular metal electrodes. The metal electrodes may be platinum-iridium electrodes. The first conductor wire 1211 passes through the electrode tube of the second electrode 112. The metal wire 122 constituting the first conductor wire 1211 extends distally through the electrode tube of the first electrode 111 under an exposed state and then continues to extend distally and is bent to form the fixation hook 113. The exposed metal wire 122 is electrically connected to the first electrode 111. The exposed wire 122 at the distal end of the second conductor wire 1212 extends into the electrode tube of the second electrode 112 and is electrically connected to the second electrode 112. Further, the first conductor wire 1211 between the first electrode 111 and the second electrode 112 is provided with an insulating buffer sleeve 114 to prevent a short circuit between the first electrode 111 and the second electrode 112 and improve electrical safety.

The distal end of the first conductor wire 1211 and the distal end of the second conductor wire 1212 are both exposed metal wires 122. The metal wire 122 at the distal end of the second conductor wire 1212 is connected to the second electrode 112 by welding or crimping. The metal wire 122 at the distal end of the first conductor wire 1211 is connected to the first electrode 111 by welding or crimping.

### Example 4

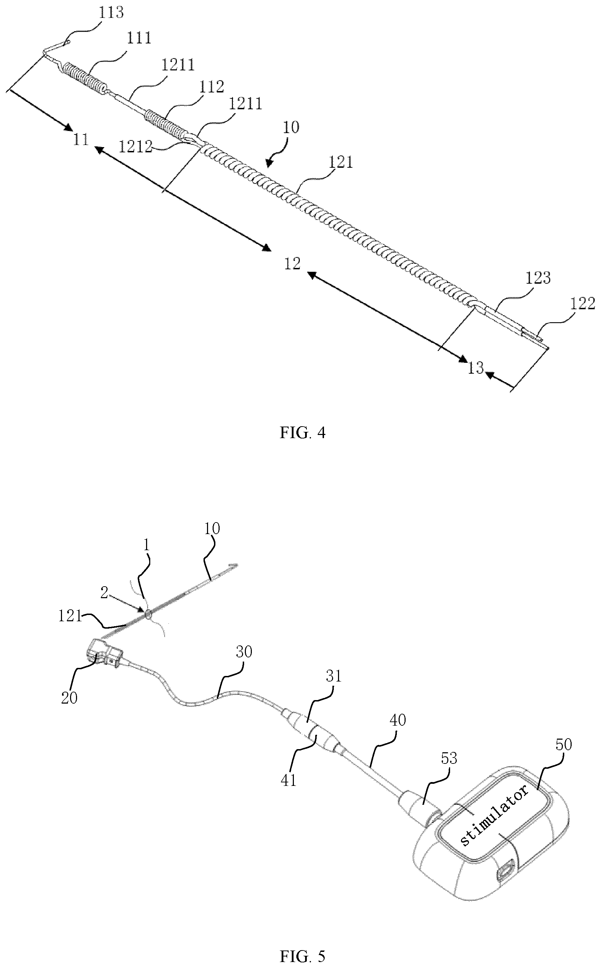

Referring to Fig. 4, the percutaneous implanted lead 10 provided in this example has two electrodes and is a bipolar electrode structure, with the difference from Example 3 in the structure of the first electrode 111 and the second electrode 112. The second electrode 112 in the present example is formed by the metal wire 122 constituting the second conductor wire 1212 extending distally and spirally wound under an exposed state. After the distal end of the first conductor wire 1211 passes through a cavity formed spirally by the second electrode 112, the first electrode 111 is formed by the metal wire 122 constituting the first conductor wire 1211 extending distally and spirally wound under an exposed state. The metal wire 122 at the distal end of the first electrode 111 continues to extend distally and is bent to form the fixation hook 113. The percutaneous implanted lead 10 of this structure is formed by winding two wires 122 with insulating layers 123, and two conductor wires 121 are alternately wound into one along the same central axis. The first electrode 111 is formed by tightly winding the metal wire 122 with the insulating layer being removed from the distal end of the first conductor wire 1211 or the directly exposed metal wire 122. The second electrode 112 formed by tightly winding the metal wire 122 with the insulating layer 123 being removed from the distal end of the second conductor wire 1212 or the directly exposed metal wire 122 on the first conductor wire 1211 having the insulating layer 123. Since the second electrode 112 is wound on the insulating layer 123 of the first conductor wire 1211, the second electrode 112 is not electrically connected to the first electrode 111. The first electrode 111 and the second electrode 112 may perform independent electrical stimulation functions, respectively, greatly enriching the diversity of treatment.

At present, the spinal percutaneous implanted lead on the market has a minimum diameter of 1.27 mm. The percutaneous implanted lead needs a relatively thick 14G puncture needle (G is the specification of Birmingham Wire Gauge (BWG); the bigger "G", the thinner the outer diameter of needle tube). Such thick needles are difficult to implant in peripheral nerves. Thus, the clinical need for peripheral nerves is to design the percutaneous implanted lead 10 to be 0.70 mm in diameter, which is a size range that is challenging and difficult to design and manufacture. Within this diameter range of the percutaneous implanted lead 10, an 18G needle may be designed based on the syringe structure to effectively and smoothly deliver or implant the percutaneous implanted lead 10 around the painful nerve desired by the physician. Therefore, it is also a great challenge to design the two-stranded spiral conductor wire 121 with a limited cross-sectional area and resisting fatigue distortion and bending, and insulation between the two-stranded conductor wire 121. The conductor wire 121 provided in this example is selected from stainless steel wire or cables. MP35N non-magnetic nickel-cobalt-chromium-molybdenum alloy wire or cables is convoluted to form an elastic elongated spiral conductor wire 121. The cable is formed by twisting multiple strands of stainless steel wire and may have better fatigue resistance. The elasticity and flexibility of the helical conductor wire 121 may overcome muscle tension and compression in vivo. The material design of the insulating layer 123 of the two-strand conductor wire 121 includes a polymer material PU, PTFE or ETFE to be extruded into a tube type and applied to the outer layer of the metal wire 122, or the polymer material PU, PTFE or ETFE sprayed onto the metal wire 122 by the coating process to form the insulating layer 123, so as to ensure the insulation between the first conductor wire 1211 and the second conductor wire 1212. Both the extrusion and spraying processes need to ensure that the insulation wall is uniform, so as to ensure the insulation performance between the first conductor wire 1211 and the second conductor wire 1212 during the implantation treatment. The diameter of the metal wire 122 is preferably 0.1 mm-0.15 mm. The outer diameters of the first electrode 111 and the second electrode 112 are preferably 0.55 mm-0.70 mm. In the peripheral nerve stimulation system provided in this example, although the bipolar percutaneous implanted lead 10 is used, since the first electrode 111 and the second electrode 112 are coaxially spaced with equal diameter, the first conductor wire 1211 and the second conductor wire 1212 are insulated and then wound into an open-type insulating spiral structure. The percutaneous implanted lead 10 with the first electrode 111 and the second electrode 112 may be implanted into the body using an implantation tool (puncture needle) with the same diameter size of the existing similar products, which is also less invasive but may provide a more effective treatment for nerve regulation.

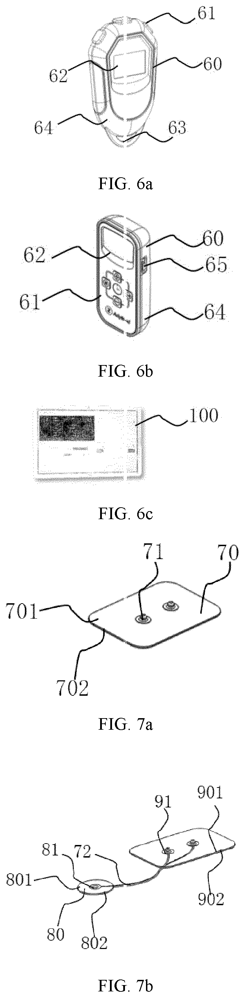

Referring to FIGS. 5, 6a-6c, 7a and 7b, the peripheral nerve stimulation system using the percutaneous implanted lead 10 provided in the present disclosure includes a percutaneous implanted lead 10, an adapter 20, a first cable 30, a stimulator 50, a remote controller 60 and a surface electrode 70. The adapter 20 is electrically connected to the proximal end of the percutaneous implanted lead 10. Further, the conductor wire 121 constituting the lead body 12 may be linearly extended to the proximal end to form the lead tail end 13. Specifically, the adapter 20 is electrically connected to the metal wire 122 constituting the lead body 12. The adapter 20 may establish a reliable connection with the inner metal wire 122 after penetrating the insulating layer 123 of the conductor wire 121. The connection position may be on the lead body 12 or on the lead tail end 13. In other embodiments, the metal wire 122 at the distal end of the lead tail end 13 may be exposed and electrically connected to the adapter 20. The adapter 20 is electrically connected to the stimulator 50 via the first cable 30, and the stimulator 50 is used for sending electrical stimulation pulses. The surface electrode is electrically connected to the stimulator 50 and forms a loop with the percutaneous implanted lead 10. As shown in Fig. 7a, in an embodiment, the surface electrode is a fixed surface electrode 70 which is fixed on the bottom of the stimulator 50 and is electrically connected to the stimulator 50 via a first surface electrode interface 71. Specifically, the fixed surface electrode 70 is composed of a first surface patch 701, a hydrogel 702 and the first surface electrode interface 71. One side of the first surface patch 701 is connected to the stimulator 50. The hydrogel 702 is coated on the first surface patch 701 so as to adhere the whole stimulator 50 to the human skin. The first surface electrode interface 71 is disposed on the first surface patch 701 and is in communication with the hydrogel 702, which is conductive to form a conductive layer. Meanwhile, the stimulator 50 is provided with an electrode interface. The fixed surface electrode 70 and the stimulator 50 are electrically and mechanically connected via the surface electrode interface 71 and the electrode interface on the stimulator 50, so that the first surface patch 701 is integrated with the stimulator 50. Then the whole stimulator 50 is pasted and fixed on the body surface of the patient via the hydrogel 702 on the surface thereof. The stimulator 50, the percutaneous implanted lead 10 and the hydrogel 702 form a loop in the human body to constitute a pathway for achieving electrical stimulation. Alternatively, in another embodiment, as shown in Fig. 7b, the surface electrode is a movable surface electrode 80 including a second surface patch 801, a second surface electrode interface 81 and a hydrogel 802. The hydrogel 802 is coated on the second surface patch 801 to enable the movable surface electrode 80 to be attached to the human skin. The second surface electrode interface 81 is disposed on the second surface patch 801 and is in communication with the hydrogel 802. The hydrogel 802 forms a conductive layer. In this example, it further includes a third surface patch 901. A third surface electrode interface 91 is disposed on the third surface patch 901. The third surface patch 901 is coated with an adhesive 902 which is a skin-friendly adhesive and is non-conductive. The whole stimulator 50 is adhered to the human skin via the adhesive 902. A patient or a doctor may choose to connect the movable surface electrode 80 with the stimulator 50 via the second cable 72. Specifically, one end of the second cable 72 is connected with the second surface electrode interface 81, and the other end of the second cable 72 is connected with the third surface electrode interface 91. The third surface electrode interface 91 is electrically and mechanically connected with the electrode interface on the stimulator 50, so that the third surface patch 901 is integrated with the stimulator 50. Then, the whole stimulator 50 is pasted and fixed on the patient's body surface by the adhesive 902 on the surface thereof. The stimulator 50, the percutaneous implanted lead 10 and the hydrogel 802 form a loop in the human body, constituting a pathway for realizing electrical stimulation. The position of the movable surface electrode 80 may be freely moved, so that the electrical performance is optimal. By exiting the second cable 72, the movable surface electrode 80 may be placed anywhere the patient requires stimulation, such as where it is most sensitive to stimulation, so as to more effectively suppress pain. Further, it also includes a third cable 40, the first cable 30 and the third cable 40 being detachably connected, and the third cable 40 being detachably connected to the stimulator 50. Specifically, the proximal end of the first cable 30 has a first connection port 31. The distal end of the third cable 40 has a second connection port 41. The first connection port 31 and the second connection port 41 are matched with the detachable connection. The proximal end of the third cable 40 has a third connection port 53, the third connection port 53 being detachably connected to the stimulator 50 for convenient use. In other embodiments, the first cable 30 and the third cable 40 are non-removably and integrally connected. Whether the cables are detachably connected or not is set as needed, and the present disclosure is not limited thereto.

The specific working process and principle are as follows. After the tiny percutaneous implanted lead 10 is implanted near the peripheral nerve related to pain suppression, the proximal end of the percutaneous implanted lead 10 exposed from the human tissue is connected to the stimulator 50 via the micro-adapter 20. The stimulator 50 forms a loop with the percutaneous implanted lead 10 by the surface electrode 70 and the percutaneous implanted lead 10. The stimulator 50 sends an electrical stimulation pulse required for treatment, so as to achieve the purpose of nerve regulation. The stimulation pulse may be controlled by the remote controller 60 to select different stimulation parameters, such as pulse width, frequency and amplitude. The percutaneous implanted lead 10 is a bipolar electrode structure, which may further improve the clinical therapeutic effect achieved by current unipolar electrode stimulation.

With continued reference to Figs. 6a, 6b and 6c, the patient selects the stimulation program and stimulation intensity prescribed by the physician via a remote controller 60 which is in wireless communication with the stimulator 50. Specifically, in an example, as shown in Fig. 6a, the remote controller 60 includes a hand grip 64, preferably sized and shaped to allow the patient to grasp with one hand, a control key 61 disposed at an end of the hand grip 64 to facilitate one-handed operation of the patient, a display screen 62 and a hanging hole 63. The hanging hole 63 is disposed at a tail portion of the hand grip and is hung and stored when the patient is not in use. The display screen 62 is provided at the top surface of the hand grip 64 for displaying stimulation parameters and the like. The remote controller 60 stores a stimulation program sequence number and a stimulation intensity sequence number corresponding to the contents of a program table and an intensity table in a hardened memory of the stimulator 50, respectively. In another embodiment, as shown in Fig. 6b, the remote controller 60 includes a hand grip 64, a control key 61 and a display screen 62. The size and shape of the hand grip 64 are preferably able to be gripped by a single hand of a patient. The control key 61 and the display screen 62 are both provided on the top surface of the hand grip 64 for the remote control operation of a single hand of a patient. Further, the side surface of the hand grip 64 is provided with a USB interface 65 for charging the remote controller 60. Further, as shown in Fig. 6c, the stimulator 50 may be controlled by the clinical programmer 100, which sets stimulation parameters and stimulation programs for the stimulator 50 by installing a stimulation programming application program on a mobile terminal, which may be a mobile phone, a tablet computer, etc.

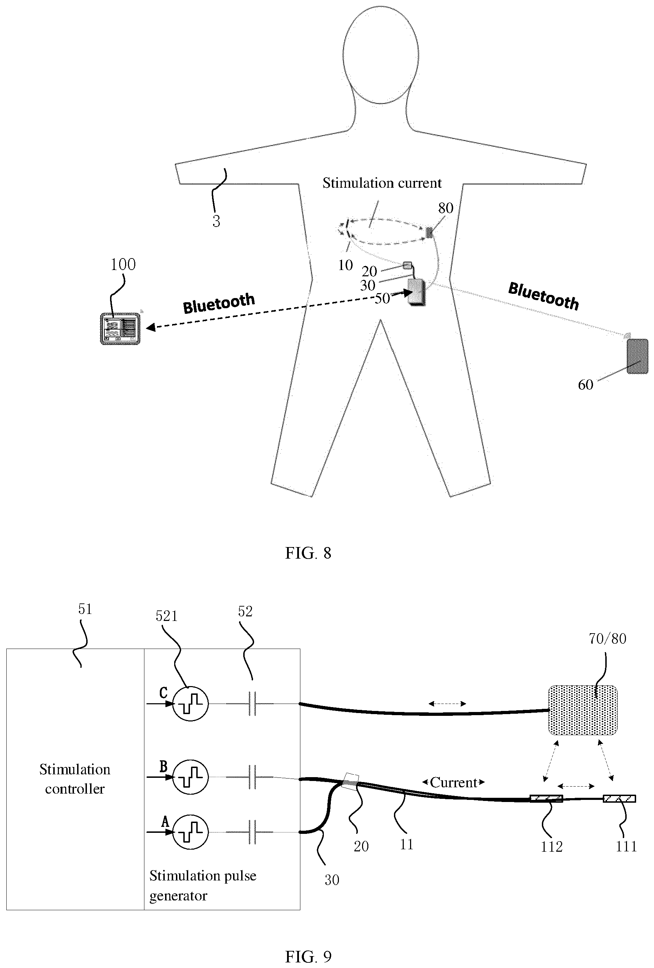

Referring to Fig. 8, according to the peripheral nerve stimulation system of the percutaneous implanted lead 10 according to the present disclosure, taking the movable surface electrode 80 and the percutaneous implanted lead 10 as an example, when in use, the percutaneous implanted lead 10 having the first electrode 111 and the second electrode 112 is percutaneously implanted in the vicinity of the target nerve in the body of the patient 3. The adapter 20 is placed on the skin of the patient 3. The stimulator 50 is fixed to the body surface of the patient 3. The movable surface electrode 80 is placed at a place where the patient 3 needs stimulation via the second cable 72. During the treatment, the patient 3 selects the stimulation program and stimulation intensity by the remote controller 60. The stimulation treatment is performed on the target site by the stimulator 50. In addition, the system includes a clinical programmer 100 implemented by the physician using a tablet computer. During the electrode implantation, the physician determines the integrity and function of the electrode by operating the control interface of the clinical programmer 100. In another embodiment, the physician debugs and determines the stimulation scheme (program) by the programming interface of the clinical programmer 100, including waveform, modulation method and safe stimulation intensity range, and solidifies these parameters into the stimulator 50. The patient uses the remote controller 60 to select a certain stimulation program and stimulation intensity by selecting the number form. The Bluetooth communication transmits the control information to the stimulator 50. The stimulator 50 generates a specific current pulse sequence according to the selected program, and outputs it to the target nerve tissue via the external lead, the adapter 20 and one of the implantation electrodes. The stimulation loop is composed of another percutaneous implanted lead or a designated surface electrode.

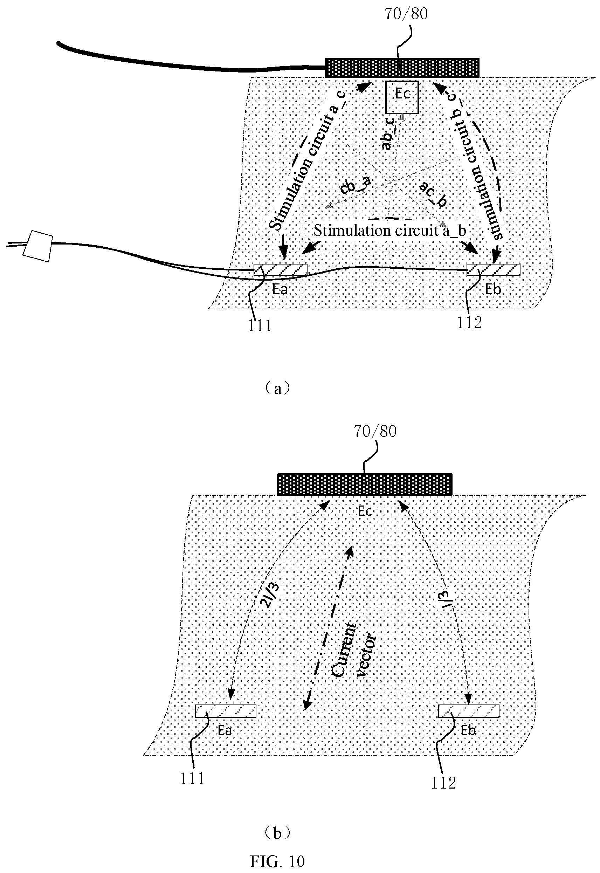

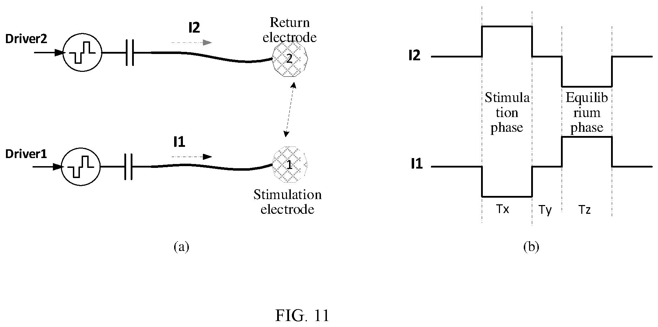

Referring to Fig. 9, the stimulator 50 is a three-channel bi-directional current pulse stimulator including a controller 51 and a pulse generator 52 including at least one pulse source 521. The controller 51 controls the output amplitude, current direction, pulse width and/or timing of the pulse source 521. The controller 51 is a microprocessor or a stimulator-specific control chip. In another example, the three-channel pulse generator 52 is implemented with three independent bi-directional current pulse sources 521 as electrode driver. Each pulse source 521 is capable of individually controlled for generation of negative (N) and positive (P) current pulses. One of the pulse sources 521 drives the surface electrode 70 or 80, and the other two drive two implanted first electrodes 111 and second electrodes 112, respectively. In this example, the output function and performance of each channel is indifferent, i.e., either one or a combination of two electrodes may be used as a stimulation electrode (negative pole) or a return electrode (positive pole), with the remaining one or two electrodes making up the stimulation return. Thus, three electrodes may form six different stimulation pathways consisting of a stimulation electrode and a return electrode, as shown in Fig. 10a. Each stimulation pathway may act on different neural tissues, or there are six possible stimulation options under the same electrode implantation conditions. Furthermore, as shown in Fig. 10b, in the case of a combination of two electrodes, by distributing the current intensity between the two electrodes, the overall flow direction of the current may be controlled, reaching the effect of the virtual electrode, thereby providing a new possibility for improving the stimulation effect. When stimulation occurs, the pulse source of the stimulation electrode and the pulse source of the return electrode form a "bridge" push-pull output mode, as shown in Fig. 11a. In the stimulation phase, the current flows from the positive pole to the negative pole, while in the equilibrium phase, the current is reversed, as shown in Fig. 11b.

In summary, the disclosure has at least the following advantages. 1. The lead body of the percutaneous implanted lead is formed into an open-type spiral structure formed by winding after the external insulation of the metal wire. Compared with the closed insulation layer formed at the periphery after the spiral winding of the metal wire, the lead body is more conducive to withstand the traction of the muscle tissue in the body and has better elasticity. Furthermore, the diameter of the percutaneous part of the lead body may be reduced to the wire diameter plus the thickness of the wrapped insulation layer, which has less trauma to human body, provides more effective treatment for neuromodulation and reduces the risk of wound infection. 2. The percutaneous implanted lead is integrally wound with one or two wires with insulation layer, so as to reduce the risk of subsequent fracture caused by processing technology in the connection process of electrical conductor and the risk of connection separation caused by fatigue wear in clinical use. 3. The lead body of the percutaneous implanted lead is designed by using extremely fine double-stranded insulated metal wire in a stacked spiral shape, and a tubular or stacked spiral double-electrode structure and a structure fixed to the human tissue are formed at the distal end of the implantation electrode. The three electrodes composed of double electrodes and a surface electrode may form six different stimulation pathways composed of stimulation electrodes and return electrodes, and each stimulation pathway may play a role in different nerve tissues. Furthermore, in the case of a combination of two electrodes, by distributing the current intensity between the two electrodes, the overall flow direction of the current is controlled, reaching the effect of the virtual electrode, providing a new possibility to increase the stimulation effect.

## Claims

1. A percutaneous implanted lead, **characterized by** comprising a distal lead head and a lead body, the distal lead head comprises a second electrode (112), a first electrode (111) and a fixation hook (113) in sequence from the proximal end to the distal end, wherein the first electrode and the second electrode are arranged along a same axial interval and insulated; the first electrode, the second electrode and a body surface electrode (70) are configured to be used in combination, the three electrodes composed of internal double electrodes and the body surface electrode may form six different stimulation pathways composed of stimulation electrodes and channel electrodes, the lead body is formed by alternate spiral winding of two conductor wires along the same central axis, wherein the two conductor wires are a first conductor wire and a second conductor wire, respectively; and the spiral turns constituting the lead body are sequentially arranged at a predetermined pitch along the axial direction, the conductor wire comprising a metal wire and an insulating layer, which is arranged outside the metal wire in a wrapping manner, such that the lead body is of an open-type insulating spiral structure;
the first electrode and the second electrode are both tubular metal electrodes; the first conductor wire is insulated to pass through the electrode tube of the second electrode; the metal wire constituting the first conductor wire extends distally through the electrode tube of the first electrode and then continues to extend distally and bend to form the fixation hook, the metal wire of the first conductor wire being electrically connected to the first electrode; the exposed metal wire of the second conductor wire at the distal end extends into the electrode tube of the second electrode and is electrically connected to the second electrode.

2. The percutaneous implanted lead according to claim 1, **characterized in that** the first conductor wire between the first electrode and the second electrode is provided with an insulating buffer sleeve.

3. The percutaneous implanted lead according to claims 1, **characterized in that** the bending angle of the fixation hook is 20-60 degrees.

4. The percutaneous implanted lead according to claim 1, **characterized in that** the metal wire has a diameter of 0.1 mm-0.15 mm, and the outer diameter of the first electrode or/and the second electrode is 0.55 mm-0.70 mm.

5. The percutaneous implanted lead according to claim 1, **characterized in that** the metal wire is 316L stainless steel wire or MP35N nonmagnetic nickel-cobalt-chromium-molybdenum alloy wire; the insulating material used for the insulating layer is polyurethane, teflon or ethylene-tetrafluoroethylene copolymer; and the insulating material is extruded to a tubular form and then applied to the periphery of the metal wire, or the insulating material is sprayed onto the metal wire by using a coating process.

6. The percutaneous implanted lead according to claim 1, **characterized in that** the exposed metal wire is welded or crimped to the first electrode or the second electrode.

7. A percutaneous implanted lead, **characterized by** comprising a distal lead head and a lead body, the distal lead head comprises a second electrode (112), a first electrode (111) and a fixation hook (113) in sequence from the proximal end to the distal end, wherein the first electrode and the second electrode are arranged along a same axial interval and insulated; the first electrode, the second electrode and a body surface electrode (70) are configured to be used in combination, the three electrodes composed of internal double electrodes and the body surface electrode may form six different stimulation pathways composed of stimulation electrodes and channel electrodes, the lead body is formed by alternate spiral winding wherein the two conductor wires are a first conductor wire and a second conductor wire, respectively; and the spiral turns constituting the lead body are sequentially arranged at a predetermined pitch along the axial direction, the conductor wire comprising a metal wire and an insulating layer, which is arranged outside the metal wire in a wrapping manner, such that the lead body is of an open-type insulating spiral structure;
the metal wire constituting the second conductor wire extends distally under an exposed state and is spirally wound to form the second electrode; the distal end of the first conductor wire is insulated to pass through a cavity spirally formed by the second electrode; the metal wire constituting the first conductor wire extends distally under an exposed state and is spirally wound to form the first electrode; and the metal wire of the first electrode at the distal end continues to extend distally and is bent to form the fixation hook.

8. The percutaneous implanted lead according to claims 7, **characterized in that** the bending angle of the fixation hook is 20-60 degrees.

9. The percutaneous implanted lead according to claim 7, **characterized in that** the metal wire has a diameter of 0.1 mm-0.15 mm, and the outer diameter of the first electrode or/and the second electrode is 0.55 mm-0.70 mm.

10. The percutaneous implanted lead according to claim 7, **characterized in that** the metal wire is 316L stainless steel wire or MP35N nonmagnetic nickel-cobalt-chromium-molybdenum alloy wire; the insulating material used for the insulating layer is polyurethane, teflon or ethylene-tetrafluoroethylene copolymer; and the insulating material is extruded to a tubular form and then applied to the periphery of the metal wire, or the insulating material is sprayed onto the metal wire by using a coating process.

## Patentansprüche

1. Perkutane implantierte Leitung, **gekennzeichnet durch** Umfassen eines distalen Leitungskopfs und eines Leitungskörpers, wobei der distale Leitungskopf eine zweite Elektrode (112), eine erste Elektrode (111) und einen Fixierungshaken (113) der Reihe nach von dem proximalen Ende zu dem distalen Ende umfasst, wobei die erste Elektrode und die zweite Elektrode entlang desselben axialen Intervalls eingerichtet und isoliert sind; wobei die erste Elektrode, die zweite Elektrode und eine Körperoberflächenelektrode (70) dazu konfiguriert sind, in Kombination verwendet zu werden, wobei sich die drei Elektroden aus internen Doppelelektroden zusammensetzen und die Körperoberflächenelektrode sechs unterschiedliche Stimulationspfade bilden kann, die sich aus Stimulationselektroden und Kanalelektroden zusammensetzen, wobei der Leitungskörper von einer alternierenden Spiralwicklung von zwei Leiterdrähten entlang derselben Mittelachse gebildet wird, wobei die zwei Leiterdrähte ein erster Leiterdraht bzw. ein zweiter Leiterdraht sind; und die Spiralwindungen, die den Leitungskörper bilden, sequenziell in einem vorbestimmten Abstand entlang der Axialrichtung eingerichtet sind, wobei der Leiterdraht einen Metalldraht und eine Isolierschicht umfasst, die außerhalb des Metalldrahts auf umhüllende Weise eingerichtet ist, so dass der Leitungskörper eine isolierende Spiralstruktur vom offenen Typ ist;
wobei die erste Elektrode und die zweite Elektrode beide röhrenförmige Metallelektroden sind; wobei der erste Leiterdraht isoliert ist, um durch die Elektrodenröhre der zweiten Elektrode hindurchzutreten; wobei sich der Metalldraht, der den ersten Leiterdraht bildet, distal durch die Elektrodenröhre der ersten Elektrode erstreckt und dann fortfährt, sich distal zu erstrecken und zu biegen, um den Fixierungshaken zu bilden, wobei der Metalldraht des ersten Leiterdrahts elektrisch mit der ersten Elektrode verbunden ist; wobei sich der freigelegte Metalldraht des zweiten Leiterdrahts an den distalen Enden in die Elektrodenröhre der zweiten Elektrode erstreckt und elektrisch mit der zweiten Elektrode verbunden ist.

2. Perkutane implantierte Leitung nach Anspruch 1, **dadurch gekennzeichnet, dass** der erste Leiterdraht zwischen der ersten Elektrode und der zweiten Elektrode mit einer isolierenden Pufferhülse versehen ist.

3. Perkutane implantierte Leitung nach Anspruch 1, **dadurch gekennzeichnet, dass** der Biegewinkel des Fixierungshakens 20-60 Grad beträgt.

4. Perkutane implantierte Leitung nach Anspruch 1, **dadurch gekennzeichnet, dass** der Metalldraht einen Durchmesser von 0,1 mm - 0,15 mm aufweist und der Außendurchmesser der ersten Elektrode und/oder der der zweiten Elektrode 0,55 mm - 0,70 mm beträgt.

5. Perkutane implantierte Leitung nach Anspruch 1, **dadurch gekennzeichnet, dass** der Metalldraht ein 316L-Edelstahldraht oder ein nichtmagnetischer MP35N-Nickel-Kobalt-Chrom-Molybdan-Legierungsdraht ist; das Isoliermaterial, das für die Isolierschicht verwendet wird, Polyurethan, Teflon oder Ethylen-Tetrafluorethylen-Copolymer ist und das Isoliermaterial in einer Röhrenform extrudiert und dann auf den Umfang des Metalldrahts aufgebracht wird oder das Isoliermaterial durch Verwenden eines Beschichtungsverfahrens auf den Metalldraht gesprüht wird.

6. Perkutane implantierte Leitung nach Anspruch 1, **dadurch gekennzeichnet, dass** der freigelegte Metalldraht an die erste Elektrode oder die zweite Elektrode geschweißt oder gecrimpt wird.

7. Perkutane implantierte Leitung, **gekennzeichnet durch** Umfassen eines distalen Leitungskopfs und eines Leitungskörpers, wobei der distale Leitungskopf eine zweite Elektrode (112), eine erste Elektrode (111) und einen Fixierungshaken (113) der Reihe nach von dem proximalen Ende zu dem distalen Ende umfasst, wobei die erste Elektrode und die zweite Elektrode entlang desselben axialen Intervalls eingerichtet und isoliert sind; wobei die erste Elektrode, die zweite Elektrode und eine Körperoberflächenelektrode (70) dazu konfiguriert sind, in Kombination verwendet zu werden, wobei sich die drei Elektroden aus internen Doppelelektroden zusammensetzen und die Körperoberflächenelektrode sechs unterschiedliche Stimulationspfade bilden kann, die sich aus Stimulationselektroden und Kanalelektroden zusammensetzen, wobei der Leitungskörper von einer alternierenden Spiralwicklung gebildet wird, wobei die zwei Leiterdrähte ein erster Leiterdraht bzw. ein zweiter Leiterdraht sind; und die Spiralwindungen, die den Leitungskörper bilden, sequenziell in einem vorbestimmten Abstand entlang der Axialrichtung eingerichtet sind, wobei der Leiterdraht einen Metalldraht und eine Isolierschicht umfasst, die außerhalb des Metalldrahts auf umhüllende Weise eingerichtet ist, so dass der Leitungskörper eine isolierende Spiralstruktur vom offenen Typ ist;
wobei sich der Metalldraht, der den zweiten Leiterdraht bildet, unter einem freigelegten Zustand distal erstreckt und spiralförmig gewickelt ist, um die zweite Elektrode zu bilden; wobei das distale Ende des ersten Leiterdrahts isoliert ist, um durch einen Hohlraum hindurchzutreten, der spiralförmig von der zweiten Elektrode gebildet wird; wobei sich der Metalldraht, der den ersten Leiterdraht bildet, unter einem freigelegten Zustand distal erstreckt und spiralförmig gewickelt ist, um die erste Elektrode zu bilden; und der Metalldraht der ersten Elektrode am distalen Ende fortfährt, sich distal zu erstrecken und zu biegen, um den Fixierungshaken zu bilden.

8. Perkutane implantierte Leitung nach Anspruch 7, **dadurch gekennzeichnet, dass** der Biegewinkel des Fixierungshakens 20-60 Grad beträgt.

9. Perkutane implantierte Leitung nach Anspruch 7, **dadurch gekennzeichnet, dass** der Metalldraht einen Durchmesser von 0,1 mm - 0,15 mm aufweist und der Außendurchmesser der ersten Elektrode und/oder der der zweiten Elektrode 0,55 mm - 0,70 mm beträgt.

10. Perkutane implantierte Leitung nach Anspruch 7, **dadurch gekennzeichnet, dass** der Metalldraht ein 316L-Edelstahldraht oder ein nichtmagnetischer MP35N-Nickel-Kobalt-Chrom-Molybdan-Legierungsdraht ist; das Isoliermaterial, das für die Isolierschicht verwendet wird, Polyurethan, Teflon oder Ethylen-Tetrafluorethylen-Copolymer ist und das Isoliermaterial in einer Röhrenform extrudiert und dann auf den Umfang des Metalldrahts aufgebracht wird oder das Isoliermaterial durch Verwenden eines Beschichtungsverfahrens auf den Metalldraht gesprüht wird.

## Revendications

1. Sonde implantée par voie percutanée, **caractérisée en ce qu'**elle comprend une tête de sonde distale et un corps de sonde, la tête de sonde distale comprend une deuxième électrode (112), une première électrode (111) et un crochet de fixation (113) en séquence de l'extrémité proximale à l'extrémité distale, dans laquelle la première électrode et la deuxième électrode sont agencées le long d'un même intervalle axial et isolées ; la première électrode, la deuxième électrode et une électrode de surface corporelle (70) sont configurées pour être utilisées en combinaison, les trois électrodes composées de doubles électrodes internes et de l'électrode de surface corporelle peuvent former six trajets de stimulation différents composés d'électrodes de stimulation et d'électrodes de canal, le corps de sonde est formé par enroulement alterné en spirale de deux fils conducteurs le long du même axe central, dans laquelle les deux fils conducteurs sont un premier fil conducteur et un second fil conducteur, respectivement ; et les spires spiralées constituant le corps de sonde sont agencées séquentiellement selon un pas prédéterminé le long de la direction axiale, le fil conducteur comprenant un fil métallique et une couche isolante, qui est agencée à l'extérieur du fil métallique d'une manière enveloppante, de sorte que le corps de sonde présente une structure en spirale isolante de type ouvert ;
la première électrode et la deuxième électrode sont toutes deux des électrodes métalliques tubulaires ; le premier fil conducteur est isolé pour passer à travers le tube d'électrode de la deuxième électrode ; le fil métallique constituant le premier fil conducteur s'étend de manière distale à travers le tube d'électrode de la première électrode puis continue à s'étendre de manière distale et se courbe pour former le crochet de fixation, le fil métallique du premier fil conducteur étant électriquement connecté à la première électrode ; le fil métallique exposé du second fil conducteur à l'extrémité distale s'étend dans le tube d'électrode de la deuxième électrode et est connecté électriquement à la deuxième électrode.

2. Sonde implantée par voie percutanée selon la revendication 1, **caractérisée en ce que** le premier fil conducteur entre la première électrode et la deuxième électrode est pourvu d'un manchon tampon isolant.

3. Sonde implantée par voie percutanée selon la revendication 1, **caractérisée en ce que** l'angle de courbure du crochet de fixation est de 20 à 60 degrés.

4. Sonde implantée par voie percutanée selon la revendication 1, **caractérisée en ce que** le fil métallique a un diamètre de 0,1 mm à 0,15 mm, et le diamètre extérieur de la première électrode et/ou de la deuxième électrode est de 0,55 mm à 0,70 mm.

5. Sonde implantée par voie percutanée selon la revendication 1, **caractérisée en ce que** le fil métallique est un fil en acier inoxydable 316L ou un fil en alliage nickel-cobalt-chrome-molybdène non magnétique MP35N ; le matériau isolant utilisé pour la couche isolante est du polyuréthane, du téflon ou un copolymère éthylène-tétrafluoroéthylène ; et le matériau isolant est extrudé en une forme tubulaire puis appliqué sur la périphérie du fil métallique, ou le matériau isolant est pulvérisé sur le fil métallique à l'aide d'un processus de revêtement.

6. Sonde implantée par voie percutanée selon la revendication 1, **caractérisée en ce que** le fil métallique exposé est soudé ou serti à la première électrode ou à la deuxième électrode.

7. Sonde implantée par voie percutanée, **caractérisée en ce qu'**elle comprend une tête de sonde distale et un corps de sonde, la tête de sonde distale comprend une deuxième électrode (112), une première électrode (111) et un crochet de fixation (113) en séquence de l'extrémité proximale à l'extrémité distale, dans laquelle la première électrode et la deuxième électrode sont agencées le long d'un même intervalle axial et isolées ; la première électrode, la deuxième électrode et une électrode de surface corporelle (70) sont configurées pour être utilisées en combinaison, les trois électrodes composées de doubles électrodes internes et de l'électrode de surface corporelle peuvent former six trajets de stimulation différents composés d'électrodes de stimulation et d'électrodes de canal, le corps de sonde est formé par enroulement alterné en spirale, dans laquelle les deux fils conducteurs sont un premier fil conducteur et un second fil conducteur, respectivement ; et les spires spiralées constituant le corps de sonde sont agencées séquentiellement selon un pas prédéterminé le long de la direction axiale, le fil conducteur comprenant un fil métallique et une couche isolante, qui est agencée à l'extérieur du fil métallique d'une manière enveloppante, de sorte que le corps de sonde présente une structure en spirale isolante de type ouvert ;
le fil métallique constituant le second fil conducteur s'étend de manière distale dans un état exposé et est enroulé en spirale pour former la deuxième électrode ; l'extrémité distale du premier fil conducteur est isolée pour passer à travers une cavité formée en spirale par la deuxième électrode ; le fil métallique constituant le premier fil conducteur s'étend de manière distale dans un état exposé et est enroulé en spirale pour former la première électrode ; et le fil métallique de la première électrode à l'extrémité distale continue à s'étendre de manière distale et est courbé pour former le crochet de fixation.

8. Sonde implantée par voie percutanée selon la revendication 7, **caractérisée en ce que** l'angle de courbure du crochet de fixation est de 20 à 60 degrés.

9. Sonde implantée par voie percutanée selon la revendication 7, **caractérisée en ce que** le fil métallique a un diamètre de 0,1 mm à 0,15 mm, et le diamètre extérieur de la première électrode et/ou de la deuxième électrode est de 0,55 mm à 0,70 mm.

10. Sonde implantée par voie percutanée selon la revendication 7, **caractérisée en ce que** le fil métallique est un fil en acier inoxydable 316L ou un fil en alliage nickel-cobalt-chrome-molybdène non magnétique MP35N ; le matériau isolant utilisé pour la couche isolante est du polyuréthane, du téflon ou un copolymère éthylène-tétrafluoroéthylène ; et le matériau isolant est extrudé en une forme tubulaire puis appliqué sur la périphérie du fil métallique, ou le matériau isolant est pulvérisé sur le fil métallique à l'aide d'un processus de revêtement.
